# EUROPEAN PATENT SPECIFICATION

(11) **EP 1 569 690 B1**
(45) Date of publication and mention of the grant of the patent: **27.07.2011**
(21) Application number: 03781200.5
(22) Date of filing: 12.12.2003
(51) Int. Cl.: A61K 39/395, A61K 47/48, A61K 51/10, A61M 1/36

(54) **ANTILYMPHOMA TARGETING AGENTS WITH EFFECTOR AND AFFINITY FUNCTIONS LINKED BY A TRIFUNCTIONAL REAGENT**
ANTI-LYMPHOM-MITTEL MIT EFFEKTOR- UND AFFINITÄTSFUNKTIONEN IN VERBINDUNG MIT EINEM TRIFUNKTIONALEN REAGENS
AGENTS DE TRAITEMENT ANTI-LYMPHOME POSSEDANT DES FONCTIONS D'EFFECTEUR ET D'AFFINITE LIEES AU MOYEN D'UN REACTIF TRIFONCTIONNEL

(30) Priority: 13.12.2002 SE 0203731; 13.12.2002 US 433012 P
(43) Date of publication of application: 07.09.2005
(73) Proprietor: Mitra Medical Technology AB, 223 70 Lund (SE)
(72) Inventor: SANDBERG, Bengt, S-245 62 Hjärup (SE); NILSSON, Rune, S-226 48 Lund (SE)
(74) Representative: Henriksson, Dan Ragnar Mikael
(86) International application number: PCT/SE2003/001949
(87) International publication number: WO 2004/054615

(56) References cited:
- WO-A1-00/02050
- WO-A1-00/09160
- WO-A1-01/80884
- US-A1- 2001 023 288
- WILBUR D. SCOTT ET AL.: 'Biotin reagents for antibody pretargeting. 4. Selection of biotin conjugates for in vivo application based on their dissociation rate from avidin and streptavidin' BIOCONJUGATE CHEM. vol. 11, 2000, pages 569 - 583, XP002213666
- WEIDEN PAUL L. ET AL.: 'Pretargeted radioimmunotherapy (PRIT TM) for treatment of Non-Hodgkin's lymphoma (NHL): initial phase l/II study results' CANCER BIOTHERAPY & RADIOPHARMACEUTICALS vol. 15, no. 1, 2000, pages 15 - 29, XP000900922

## Description

### Technical Field of the Invention.

The present invention relates to a medical agent comprising

### Background Art

Lymphomas are malignant cell infiltrations of the lymphatic system. The lymphatic system includes the nodes which are located in the neck, armpit, and groin. These nodes are only part of the lymphatic system, as they are connected to each other and to the spleen, thymus, and parts of the tonsils, stomach, and small intestine by a network of vessels. The vessels carry a fluid called lymph which contains lymphocytes. Once a malignancy begins in one part of the lymphatic system, it often spreads throughout the rest of the lymphatic system before it is detected.

There are precise, internationally agreed criteria to define the stage of disease for each type of cancer. For lymphomas this means mapping out how many lymph nodes are affected. It also means finding out if the lymphoma has spread outside the lymphatic system to other organs.
Stage I: Cancer limited to one group of lymph nodes or a single organ or site outside the lymphatic system
Stage II: Cancer in two or more groups of lymph nodes all on the same side of the diaphragm
Stage III: Cancer on both sides of the diaphragm but not outside the lymphatic system
Stage IV: Widespread cancer outside the lymphatic system

Lymphomas are divided into many sub-groups according to cell types. Generally, they are classified as non-Hodgkin's and Hodgkin's. Currently, Hadgkin' s lymphoma is more-curable than non-Hodgkin's. Non-Hodgkin's lymphomas

on average 1.5 to 3.5 reagents conjugated to an anti-CD20 antibody, wherein each reagent is 3-(13'-thioureabenzyl-(DOTA) trioxadi-amine-1-(13'-biotin-Asp-OH)trioxamine-5-isothio-cyanato-aminoisophtalate, and wherein the anti-CD20 antibody is rituximab, as well as to a

kit for extracorporeal elimination or at least reduction of the concentration of said medical agent as a non-tisssue bound therapeutic or diagnostic medical agent in the plasma or whole blood of a mammalian host, said kit comprising
a) said medical 1 agent, and
b) an extracorporeal device comprising an immobilised receptor to which the biotin molecule
   in the medical agent adheres. are derived from both B-cells and T-cells origins, where 90% of all cases are B-cell derived and the remaining 10% are of T-cell derivation.

The treatment for all types of lymphoma depends on the type, stage, and grade of disease. The stages and grades are outlined below.

### Stages:

I: cancer site, no bone marrow involvement
II: two sites, both either above or below the diaphragm; no bone marrow involvement
III: sites above and below the diaphragm; no bone marrow involvement
IV: bone marrow is affected or the cancer cells have spread outside the lymphatic system

### Grades:

high: usually found in B-cell and T-cell types intermediate: usually found in B-cell and T-cell types
low: predominantly found in B-cell types

Lymphomas are usually treated by a combination of chemotherapy, radiation, surgery, and/or bone marrow transplants. The cure rate varies greatly depending on the type of lymphoma and the progression of the disease.

Because lymph tissue is found in many parts of the body, non-Hodgkin's lymphoma can start in almost any part of the body. The cancer can spread to almost any organ or tissue in the body, including the liver, bone marrow, spleen, and nose.

Based on the histology, non-Hodgkin's lymphomas are divided into two groups: indolent lymphomas, which grow more slowly and have fewer symptoms, and aggressive lymphomas, which grow more quickly.

Lymphomas include follicular small cleaved cell lymphoma, adult diffuse mixed cell.lymphoma, follicular mixed cell lymphoma, adult diffuse large cell lymphoma, follicular large cell lymphoma, adult immunoblastic large cell lymphoma, adult diffuse small cleaved cell lymphoma, adult lymphoblastic lymphoma, small lymphocytic (marginal zone) adult small non-cleaved cell lymphoma.

Other types of indolent non-Hodgkin's lymphoma/- leukemia are lymphoplasmacytoid lymphoma, monocytoid B-cell lymphoma, mucosa-associated lymphoid tissue (MALT) lymphoma, splenic marginal zone lymphoma, hairy cell leukemia, and cutaneous T- cell lymphoma (Mycosis fungoides/Sezary syndrome).

Other types of aggressive non-Hodgkin's lymphoma are anaplastic large-cell lymphoma, adult T-cell lymphoma/- leukemia, mantle cell lymphoma, intravascular lymphoma-tosis, angioimmunoblastic T-cell lymphoma, angiocentric lymphoma, intestinal T-cell lymphoma, primary mediastinal B-cell lymphoma, peripheral T- cell lymphoma, lymphoblastic lymphoma, post-transplantation lymphoprolifera-tive disorder, true histiocytic lymphoma, primary central nervous system lymphoma, and primary effusion lymphoma. Aggressive lymphomas are also seen more frequently in patients who are HIV-positive (AIDS-related lymphoma).

Recurrent adult non-Hodgkin's lymphoma may come back in the lymph system or in other parts of the body.

Indolent lymphoma may come back as aggressive lymphoma. Aggressive lymphoma may come back as indolent lymphoma.

Non-Hodgkin's lymphomas (NHLs) are the fifth leading cause of cancer morbidity and mortality ( Wingo P, Tong T, Bolden S. Cancer statistics, 1995. CA Cancer J. clin 1995; 45, 8-30 & Parker SL, Tong T, Bolden S., Wingo PA. Cancer statistics, 1996. CA Cancer J. Clin 1996;46:5-27). Over the past two decades, the prevalence in the USA of these lymphomas has increased rapidly. Five years ago more than 52,000 new diagnosis were made, and 23.000 deaths were attributed to NHLs and with an incidence increasing at a rate of 7% per year (Parker SL, Tong T, Bolden S., Wingo PA. Cancer statistics, 1996. CA Cancer J. Clin 1996;46:5-27). This represents an increase of 150% in the population-adjusted new cases of NHLs over the past 50 years.

The overwhelming majority of patients (about 80%) constitute patients with NHLs of B-cell origin (Harris N.L., Jaffe E.S., Stein H. et.al.. Lymphoma classification proposal: clarification [letter]. Blood 1995; 85: 857-860 ). Despite the use of various combined chemotherapeutic regimens for advanced-stage intermediate- and high grade lymphomas, roughly half of patients treated do not have a complete remission or finally have a relapse after remission. The situation has not improved noticeably in almost two decades ( Gordon LI, Harrington D, Andersen J, et.al. N. Engl. J. Med. 1992;327:1342-9 & Fisher RI, Gaynor ER, Dahlberg S, et.al. N. Engl. J. Med. 1993; 328: 1002-6).

Treatment with standard-dose salvage chemotherapy rarely results in durable remissions and often has serious toxicity. Although the use of high-dose chemotherapy with bone marrow transplantation has shown to be promising, not all patients derive long-term benefits from this type of treatment (Armitage JO. Blood 1989;73:1749-58). A curative treatment for patients with advanced low-grade lymphoma still remains to be clearly established (DeVite VT Jr., Jaffe ES, Mauch P, Longo DL. Lymphocytic lymphomas. In: DeVita VT Jr., Hellman S., Rosenberg SA. Eds. Cancer: principles and practice of oncology. 3rd ed. Vol.2. Philadelphia: J.B. Lippincott, 1989;1741-98). Treatment with anthracycline-based chemotherapy regimes results in complete remission in 50-90 percent of patients with intermediate and high-grade non-Hodgkin's lymphoma and long-term disease-free survival in 30-60 percent.

Unfortunately, few patients with low-grade lymphoma or relapses of any type of lymphoma can be cured with conventional approaches (Armitage JO. N.Engl.J.Med. 1993; 328:1023-30). High-dose chemoradiotherapy with bone marrow transplantation cures 10-50% of patients with lymphoma in relapse, but 40-80% relapse again and 5-20% die of complications related to transplantation (Appelbaum FR, Sullivan KM, Buckner CD et.al. J.Clin. Oncol. 1987;5:1340-7 & Freedman AS, Takvorian T, Anderson KC et.al. J.Clin.Oncol. 1990;8:784-91). The use of large doses of chemoradiotherapy has not been feasible because of unacceptable morbidity and mortality (Bearman SI, Appelbaum FR, Bruchner CD. et.al. J.Clin.Oncol. 1988; 6:1562-8).

Tissue or organ specific localisation of a medical agent is a very important factor in its effective application. Lack of specific tissue localisation is of pati-cular importance in the treatment with cytotoxic agents, where the desired effect is to kill certain types of cells, such as in the treatment of cancer.

The treatment of cancer with agents specific for the tumour cell without harming the host has long been a.goal of oncology. The development of monoclonal antibodies provided hope that tumour-targeted therapy would one day play a role in the treatment of cancer. Indeed, promising results have been presented in several areas; however, most of the treatment modalities have often proved technically difficult, produced disappointing efficacy, and were often not broadly applicable to patients with a given malignancy.

The treatment of patients with lymphoma is an exception. Patients with advanced stage or relapsed low-grade non-Hodgkin's lymphoma (NHL) are not curable using conventional approaches and are usually treated with combination chemotherapy regimens of increasing intensity as needed to reduce disease and palliate symptoms. Recent attempts utilising supralethal chemotherapy combined with radiotherapy followed by bone marrow transplantation have resulted in an approximately 20% long term disease-free survival rate (F. Applebaum et al, J. Clin.Oncol. 5:1340, 1987). However, most patients treated in this manner die of lymphoma or treatment complications. Therefore, new strategies for the treatment of non-Hodgkin's lymphomas are needed. These strategies should be aiming at the maximisation of therapeutic effect coupled with the minimization of toxicity.

One approach involves the use of monoclonal antibodies that recognise tumour-associated antigens as a means of targeting drugs or radioisotopes to tumour cells. This approach is particularly attractive in the case of NHL as the lymphoma tumour cells display a variety of tumour-restricted antigens on their cell surfaces that would be available for targeting (A.J. McMichael, Leukocyte Typing III, pp 302-363 and 432-469, Oxford University Press, Oxford, England, 1987). The rationale for utilising such an approach is further supported by the observation that monoclonal antibodies by themselves can exhibit anti-tumour effects in vivo. Of all the malignancies that have been treated with monoclonal antibodies to date, the lymphomas have yielded the most dramatic results. Significant tumour regressions have been reported in patients treated with monoclonal anti-idiotype antibodies (R.A Miller et, New Eng. J. Med. 306:517, 1982; T.C. Meeker et al, Blood 65:1349, 1985). Most of the tumour responses, however, have been incomplete and of relatively short duration. The practical problem of generating anti-idiotype antibodies restricts the utility of such an approach (T. Meeker et al, New. Eng. J. Med 312:1658, 1985).

Recently, a number of monoclonal antibodies have been developed which recognise antigenic sites on both malignant and normal human B cells. These pan B-cell antibodies have been useful in classifying lymphomas and in defining the ontogeny and biology of normal B cells. Because of the limited efficacy of unmodified antibodies in general, recent attention has focused on the use of antibodies conjugated to cytotoxic agents. Among the cytotoxic agents that might be considered, radioisotopes are especially attractive, as lymphomas are especially sensitive to the effects of radiation. Moreover, such radiolabelled antibodies may be of considerable utility in terms of diagnostic imaging of tumour involved sites. Most of these cytotoxic anti-lymphoma antibodies are directed towards CD20.

CD20 is an antigen that is a 35 kilodaltons, non-glycoylated phosphoprotein found on the surface of greater than 90% of B cells from peripherial blood or lymphoid organs. The antigen is expressed on the surface of virtually all resting B cells maintained in culture, but is lost by approximately one-third of the population upon activation of the cells by protein A or exposure to Epstein-Barr virus. This result has been interpreted to mean that CD20 is lost during terminal differentiation of B cells (L. M. Nadler, Lymphcyte typing II, vol 2 pp 3-37 and 65 Appendix, E. L. Renling et al eds Springer Verlag, 1986).

A number of other antigens like the CD19 are also expressed on the surface of cells of the B lineage. However, contrary to the CD20, antibodies binding to the CD19 are rapidly internelised. Other antibodies identified as binding to these types of cells are: the B2 binding to the CD21 antigen; B3 binding to the CD22 antigen and the J5 binding to the CD 10 antiden. The pan-B-cell antibody MB-1 is also of interest and has been shown to bind to CD37.

Naked antibodies directed against CD20 have shown to have efficiency. One registered naked antibody, Rituximab, is a chimeric mouse/human anti-CD20 antibody that has shown efficiency in the treatment of indolent lymphoma, especially follicular lymphoma. The overall response rate for patients with indolent lymphoma is 50% and the complete response rate is 10% (McLaughlin P et al, J. Clin. Oncol. 16: 2825-2833, 1998.). Time to progression has been reported to be 13 months. Rituximab does also produce objective remissions in aggressive lymphoma albeit with a lower response rate. Nonetheless virtually all patients treated with Rituximab as a single agent will finally relapse.

Systemic radiotherapy is an established form of treatment. The use of radioiodine in the treatment of disseminated cancer of the thyroid is often the mainstay of therapy. Radioimmunotherapy (RIT) is another form of systemic radiotherapy where the radionuclide is targeted by an antibody to a tumour cell. RIT is in some cases a combined modality between radiotherapy and immunotherapy, since the antibody itself may exert an anti-tumour effect. The use of RIT is still experimental, but several encouraging studies have been published. In treatment of B-cell lymphoma several groups have reported long term remissions following RIT. Most investigators have used ¹³¹I or ⁹⁰Y labelled mouse antibodies directed to the CD20 antigen (Kaminski, M. S. et al, J. Clin. Oncol., 14:1974 -1981, 1996, Knox, S. J et al, Clin. Cancer Res., 2: 457-470, 1996.).

Therapeutic application of chimeric and radiolabelled antibodies for treatment of B cell lymphoma is described by Anderson, D.R. et.al. in EP 0 752 248 B1; EP 669 836 B1 and US 5,843,439; 5,776,456; 5,736,137. Methods for the treatment of lymphoma by administration of a B cell-specific antibody are described in Kaminski, M.S. et.al. US 5,595,721; 6,015,542; 5,843,398; 6,090,365 and by Goldenberg, D.M. et.al. in US 6,183,744 B1. Other patents and patent applications related to the subject matter are US 6,399,061 B1, EP 1 005 870 A2, WO 98/42378, WO 99/57981, WO 00/09160, WO 00/27428, WO 00/27433, WO.01/34194 A1, WO 01/10462 A1, WO 01/10460 A1, WO 00/67795, WO 00/52473.

Rituximab is a chimeric mouse/human antibody that has been engineered from its mouse parental antibody, ibritumomab. When ibritumomab is labelled with ⁹⁰Y, it is entitled Zevalin™. Wiseman et.al. Critical reviews in Oncology/Hematology 39 (2001), 181-194, have reported that Zevalin™ may be administered safely without prior dosimetry at an activity of 15 MBq/kg to patients with a platelet count of > 149 x 10⁹ /L. For patients with platelet counts of 100-149 x 10⁹, an activity of 11.1 MBq/ kg is well tolerated. A prospective randomised trial of Zevalin in patients with relapsed or refractory indolent or transformed lymphoma compared to a standard course of Rituximab has been reported. Among 143 patients studied, an overall response of 80% was found for the Zevalin group vs 56% in the group who received unlabelled Rituximab (P=0.002) and with 30% complete remission with Zevalin vs 16% CR for Rituximab (P=0.04) Zevalin has also been evaluated in patients with follicular lymphoma refractory to Rituximab. The response duration was significantly longer (8.4+ vs 4 months) for Zevalin as compared with prior Rituximab (P=0.008).

Tositumomab is a murine IgG₂ₐ lambda monoclonal antibody directed against the CD20 antigen. I¹³¹-tositumomab (Bexxar^{®}) is a radio-iodinated derivative of tositumomab that has been covalently linked to Iodine-131. Iodine-131 decays with beta and gamma emissions with a physical half-life of 8.04 days. Possible mechanisms of action of the I¹³¹-tositumomab therapeutic regimen include induction apoptosis, complement dependent cytotoxicity (CDC) (and antibody-dependent cellular cytotoxicity (ADCC) mediated by the antibody) (Cardarelli PM et. al. Cancer Immunol Immunother. 2002 Mar; 51(1): 15-24; Stashenko P, et. al. J Immunol 1980; 125:1678-85). Additionally, cell death is associated with ionizing radiation from the radioisotope.

The therapeutic regimen is administered in two discrete steps: the dosimetric and the therapeutic step. Each step consists of a sequential infusion of tositumomab, followed by I¹³¹-tositumomab.

The maximum dose of the I¹³¹-tositumomab therapeutic regimen that was administered in clinical trials was 88 cGy. Three patients were treated with a total body dose of 85 cGy of Iodine I¹³¹-tositumomab in a dose escalation study. Two of the 3 patients developed grade 4 toxicity of 5 weeks duration with subsequent recovery. In addition, accidental overdose of the therapeutic regimen occurred in one patient at total body doses of 88 cGy.

Normal organ toxicity limits the amount of activity that can be safely administered to patients and thereby the absorbed dose to tumour. The first dose-limiting organ is the bone marrow. Localised B-cell lymphoma may be cured by external beam radiotherapy with a dose of 30 to 44 Gy. The dose that may be achieved with conventional radioimmunotherapy without the use of stem cell support is substantially lower. Wiseman et al has reported a median dose of 15 Gy in B-cell lymphoma in a phase III trial (Wiseman G et al., Critical reviews in Oncology/- Hematology 39 (2001) 181-194). The response rate was 80% objective response and 34% complete response. The Seattle group using stem cell support has reported the highest remission rate 80% complete remissions (Liu Steven Y. et al., J. Clin. Oncol.16(10): 3270-3278, 1998). They estimated tumour sites to achieve 27 to 92 Gy.

The non-haematological dose-limiting toxicity was reversible pulmonary insufficiency, which occurred at doses ≥ 27 Gy to the lungs. Although the studies are not quite comparable, they indicate a dose effect relationship in RIT. If there is a dose relationship, it may be possible to increase efficacy if a higher dose to the tumour can be delivered. This may be most clinically relevant, since complete remission following RIT has been associated with longer duration of remission (Wahl et al., J.Nucl. Med.39:21S-26S, 1998.).

An obstacle to this is the radio sensitivity of the bone marrow. A higher absorbed dose to the bone marrow may cause myeloablation. Thus, the dose necessary to reach a more effective therapy is hampered by the accumulation of radioactivity in the blood circulation, leading to toxicity of normal organs, such as bone marrow. Various means for clearing blood from cytotoxic targeting biomolecules (e.g. therapeutic or diagnostic monoclonal antibodies) after intravenous administration have been reported (See review article by Schriber G.J. and Kerr D. E., Current Medical Chemistry 2:616-629, (1995)).

In the so-called avidin chase modality, avidin or streptavidin is administered systemically after administration of the therapeutic or diagnostic antibody to which biotin has been attached, at a time when a sufficient amount of the antibody has been accumulated in the tumour. Avidin or streptavidin will associate with the antibodies and the so formed immunocomplex will clear from the blood circulation via the reticuloendothelial system(RES) and be cleared from the patient via the liver. These procedures will improve the clearance of biotinylated cytotoxic antibodies. An alternative approach to the same end is the use of anti-idiotypic antibodies. However, all these methods rely on the liver or kidney for blood clearance and thereby expose either or both of these vital organs as well as the urinary bladder to a high dose of cytotoxicity.

Another major drawback of the methods is the immunogenicity of these agents, particularly the streptavidin, which prevent repetitive treatments once the immune response has been developed. Extracorporeal techniques for blood clearance are widely used in kidney dialysis, where toxic materials build up in the blood due to the lack of kidney function. Other medical applications, whereby an extracorporeal apparatus can be used, include: removal of radioactive materials; removal of toxic levels of metals, removal of toxins produced from bacteria or viruses; removal of toxic levels of drugs, and removal of whole cells (e.g cancerous cells, specific haematopoietic cells - e.g. B, T, or NK cells) or removal of bacteria and viruses.

Various methods have been proposed to rapidly clear radiolabelled antibodies from blood circulation after the tumour has accumulated a sufficient quantity of immunoconjugate to obtain a diagnosis or therapy. Some of the methods employed involve enhancement of the body's own clearing mechanism through the formation of immune complexes. Enhanced blood clearance of radiolabelled antibodies can be obtained by using molecules that bind to the therapeutic antibody, such as other monoclonal antibodies directed towards the therapeutic antibody (Klibanov et al, J. Nucl. Med 29:1951-1956 (1988); Marshall et al, Br. J. Cancer 69: 502-507 (1994); Sharkey et al, Bioconjugate Chem. 8:595-604, (1997), avidin/streptavidin (Sinitsyn et al J. Nucl. Med. 30:66-69 (1989), Marshall et al Br. J. Cancer 71:18-24 (1995), or glycosyl containing compounds which are removed by receptors on liver cells (Ashwell and Morell Adv. Enzymol. 41:99-128 (1974). Still other methods involve removing the circulating immunoconjugates through extracorporeal methods (See review article by Schreiber G.J. and Kerr D.E., Current Medical Chemistry 2:616-629 (1995)).

The extracorporeal techniques used to clear a medical agent from blood circulation are particularly attractive because the toxic material is rapidly removed from the body.

Applications of these methods in the context of immunotherapy have been previously described (Henry Chemical Abstract 18:565 (1991); Hofheinze D. et al Proc. Am. Assoc. Cancer Res. 28:391 (1987); Lear J. K. et al Antibody Immunoconj. Radiopharm. 4:509 (1991); Dienhart D. G. et al Antibody Immunoconj. Radiopharm. 7:225 (1991); DeNardo S.J. et al J. Nucl. Med 33:862-863 (1992); DeNardo G.L. et al J.Nucl.Med 34:1020-1027 (1993); DeNardo G. L. J. Nucl. Med 33:863-864 (1992); and US patent No. 5,474,772 (Method of treatment with medical agents).

To make the blood clearance more effective and to enable processing of whole blood, rather than blood plasma which the above methods refer to, the medical agents (e.g. tumour specific monoclonal antibody carrying cell killing agents or radio nuclides for tumour localization) have been biotinylated and cleared by an avidin-based adsorbent on a column matrix. A number of publications provide data showing that this technique is both efficient and practical for the clearance of biotinylated and radionuclide labelled tumour specific antibodies (Norrgren K. et al, Antibody Immunoconj. Radiopharm. 4:54 (1991), Norrgren K. et al J. Nucl. Med 34:448-454 (1993); Garkavij M. et al Acta Oncologica 53:309-312 (1996); Garkavij M. et al, J. Nucl. Med. 38:895-901 (1997)).

These techniques are also described in EP 0 567 514 and US 6,251,394. The device Mitradep^{®}, developed and manufactured by Mitra Medical Technology AB, Lund, Sweden, is based on this technology. By using the avidin-coated filter in conjunction with biotin labelled therapeutic antibodies, the blood clearance technique can be applied equally well to chimeric or fully humanised antibodies. Experimental data reveal that during a three-hour adsorption procedure, more than 90% of the circulating biotinylated antibodies can be removed by the Mitradep^{®} system (Clinical Investigator's Brochure - Mitradep^{®}).

In order to be adsorbed to the extracorporeal filter, the monoclonal antibodies carrying the cytotoxic agent (e.g. radionuclide) need to be biotinylated (biotin binds irreversible to the avidin in the filter) prior to administration to the patient. The number of biotinyl moieties per IgG molecule is in the range of 3-6, typically 4.

A further development of this method with simultaneous labelling of biotin and radionuclides is described in a patent application by S. Wilbur and B.E.B. Sandberg PCT/ SE98/01345, disclosing a trifunctional reagent for the conjugation to a biomolecule.

The latter method has a number of advantages over the consequtive labeling of radio nuclides and biotinylation and is particularly attractive in cases where the naked (non-chelated) antibody is supplied to the hospital, since both the chelating group and the biotinyl groups have to be conjugated to the antibody in addition to the radiolabelling step.

However, in most cases the same type of functions (∈-amino groups) on the antibodies are utilized for coupling of the chelating groups and the biotinyl groups, leading to a competition of the most accessible sites.

Chelation and/or biotinylation of an antibody results in a heterogenous preparation. If for example a chelated antibody is determined to have 3 chelates per antibody, the preparation contains a mixture of antibodies with 1 chelate/antibody to 7 chelates/antibody. As the chelate and biotin are linked to the same moeties on the antibody, antibodies with a higher number of chelates might have a lower number of biotin. It might also results in antibodies with a high number of chelates having no biotin at all.

This means that, statistically, a population of the antibodies carrying radionuclide but not biotin will circulate in the blood, and those antibodies will not be removed by the Mitradep® filter.

To facilitate the labelling of the naked therapeutic or diagnostic antibody and to ensure that the ratio of biotin and the radiolabel is one to one, Mitra Medical Technology AB, Lund, Sweden has developed a series of novel water-soluble structures (Tag-reagent; MitraTag™) containing the two types of functions, thereby enabling simultaneous and site specific conjugation of chelating groups (for radiolabelling) and the biotin groups.

The Tag-reagent labelled with the chelating group DOTA is called MitraTag-1033.

The present invention encompasses a medical agent comprising a reagent conjugated to an anti-lymphoma antibody, and various methods for the treatment of lymphatic cancer, i.e. lymphoma, and NHL in particular.

### Summary of the Invention

The object of the present invention is to solve the above discussed problem in connection with treatment of certain lymphoma diseases. This object is achieved by the present invention as specified below.

The present invention relates in one aspect to a medical agent comprising on average 1.5 to 3.5 reagents conjugated to an anti-CD20 antibody, wherein each reagent is 3-(13'-thioureabenzyl-(DOTA)trioxadi-amine-1-(13"-biotin-Asp-OH)trioxamine-5-isothio-cyanato-aminoisophtalate, and wherein the anti-CD20 antibody is rituximab.

In a further aspect, the present invention relates to a kit for extracorporeal elimination or at least reduction of the concentration of a non-tissue-bound therapeutic or diagnostic medical agent as defined above in the plasma or whole blood of a mammalian host, said kit comprising
a) the medical agent, and
b) an extracorporeal device comprising an immobilised receptor onto which the biotin molecule in the medical agent adheres.

Further advantages and objects of the present invention will now be described in more detail with reference to the accompanying drawings.

### Brief Description of the Drawings

Fig. 1 shows depletion of 1033-rituximab conjugates during recirculation through a miniaturised Mitradep^{®}.
Fig. 2 shows a flow cytometric assay of binding to the CD20 positive cell line Raji.
Fig. 3 shows binding of 1033-conjugates to a CD20+(SB) and a CD20 - (HSB) cell line.
Fig. 4 shows competitive inhibition of ¹²⁵I-Labelled Rituximab binding to SB cells by cold rituximab and 1033-rituximab conjugates.
Fig. 5 shows whole body clearance of radioactivity in rats injected with ¹¹¹In-1033-rituximab antibody conjugates expressed as percentage ± std.dev.
Fig. 6 shows blood clearance of ¹¹¹In-1033-rituximab antibody conjugates expressed as % injected dose/gram ± std.dev.
Fig. 7 shows biodistribution of ^{III}In-1033-rituximab (4.6 1033/IgG) in rats.
Fig. 8 shows HPLC size exclusion separation of blood samples drawn from a rat injected with ^{III}In-1033-rituximab (4.6 1033/IgG).

### Description of Preferred Embodiments

With the present invention it is possible to improve the tumour to non-tumour ratio of cytotoxic targeting agents in the treatment of disseminated haematological carcinomas, in particular lymphomas, by reducing the concentration of the cytotoxic medical agent in the blood circulation after administration of a cytotoxic agent and thereby facilitating a higher dosage and hence a more effective treatment regime without exposing the vital organs to higher toxicity. Furthermore, the present invention presents new medicals and the use of these agents in the treatment of lymphatic cancer and NHL, in particular.

In one embodiment, a radiolabelled anti-lymphoma antibody is given in a single dose which is limited to what is regarded as tolerable to the patient without reconstitution of the hematopoietic function, through bone marrow transplantation, or by some other means; "low dose". The dose range will be 10-20 MBq/kg body weight of ⁹⁰Y-anti-lymphoma antibody, preferably 11-15 MBq/kg and the range for ¹¹¹In-anti-lymphoma antibody for targeting localisation will be 20-250 MBq, preferable 50-150 MBq. In this embodiment, extracorporeal clearance of non-bound radiolabelled therapeutic or diagnostic antibody is optional.

In another embodiment, a radiolabelled anti-lymphoma antibody is given in a single dose designated to deliver a high amount of radioactivity to the patient. This "high dose method" has to be combined with means of reconstituting the bone marrow or by reducing the radiation effect on bone marrow preferably by the use of the Mitradep^{®} system. For ⁹⁰Y-anti-lymphoma antibodies, "high dose" means a single dose exceeding 20 MBq/kg body weight.

In a preferred embodiment, ¹¹¹In-anti-lymphoma at a dose of 50-150 MBq is combined with a "high dose" (> 20 MBq/ kg body weight) of ⁹⁰Y-anti-lymphoma antibody, either given in sequence at intervals of 6-8 days or given simultaneously.

The following embodiments of the invention also serve to explain the details of the invention.

Lymphomas are tumours originating from lymphocytes. The normal counterparts of lymphomas, i.e. the normal lymphocytes, arise from pluripotent stem cells in the bone marrow and differentiate to fully mature lymphocytes. During their differentiation they express different cell surface antigens (CD-antigens) some of which are lineage and/or stage specific. Lymphomas can arise from lymphocytes in various differentiation stages and often present the CD-antigens expressed at this stage. These CD-antigens cannot be used only for diagnostic purposes but also as targets for different kinds of antibody therapy.

The study of human leukocyte antigens, predominantly by monoclonal antibody techniques, is a rapidly changing field of basic research and clinical investigation. Leukocyte surface molecules defined by antibodies have been assigned cluster differentiation (CD) numbers (CD-antigens) in a series of international workshops (Paris, 1982; Boston, 1984; Oxford, 1986; Vienna, 1989, Osaka, 1996). The CD classification of these antigens has become the standard form in published literature and provides a basis for standardization of clinical reporting. The current CD classification is presented in the form of a list, with a brief summary of each antigen beside each entry.

| □ **CD molecule** | **Alternate Names** | **Locus ID** | **Past Guides** |
|---|---|---|---|
| CD1a | R4; HTA1 | 909 | CD1a |
| CD1b | R1 | 910 | CD1b |
| CD1C | M241; R7 | 911 | CD1C |
| CD1d | R3 | 912 | CD1d |
| CD1e | R2 | 913 | CD1e |
| CD2 | CD2R; E-rosette receptor; T11; LFA-2 | 914 | CD2 |
| CD3delta | CD3d | 915 | |
| CD3epsilon | CD3e | 916 | |
| CD3gamma | CD3g | 917 | |
| CD4 | L3T4; W3/25 | 920 | CD4 |
| CD5 | Leu-1; Ly-1; T1; Tp67 | 921 | CD5 |
| CD6 | T12 | 923 | CD6 |
| CD7 | gp40 | 924 | |
| CD8alpha | Leu2; Lyt2; T cell co-receptor; T8 | 925 | |
| CD8beta | Leu2; CD8; Lyt3 | 926 | |
| CD9 | DRAP-27; MRP-1; p24 | 928 | CD9 |
| CD10 | EC 3.4.24.11; neprilysin; CALLA; enkephalinase; gp100; NEP | 4311 | |
| CD11a | AlphaL integrin chain; LFA-lalpha | 3683 | CD11a |
| CD11b | AlphaM integrin chain; AlphaM-beta2; C3biR; CR3; Mac-1; Mo1 | 3684 | CD11b |
| CD11c | AlphaX integrin chain; Axb2; CR4; leukocyte surface antigen p150,95 | 3687 | CD11C |
| CDw12 | p90-120 | 23444 | CDw12 |
| CD13 | APN; EC 3.4.11.2; gp150 | 290 | CD13 |
| CD14 | LPS-R | 929 | CD14 |
| CD15u | Sulphated CD15 | | |
| CD16a | FCRIIIA | 2214 | |
| CD16b | FCRIIIB | 2215 | |
| CDw17 | LacCer | | CDw17 |
| CD18 subunit; | CD11a beta subunit; CD11b beta CD11c beta subunit; beta-2 integrin chain | 3689 | CD18 |
| CD19 | B4 | 930 | CD19 |
| CD20 | B1; Bp35 | 931 | |
| CD21 | C3d receptor; CR2; EBV-R | 1380 | CD21 |
| CD22 | BL-CAM; Lyb8 933 | | CD22 |
| CD23 | B6; BLAST-2; FceRII; Leu-20; Low affinity IgE receptor | 2208 | CD23 |
| CD24 BA-1; | HSA 934 | | CD24 |
| CD25 | IL-2R alpha chain; IL-2R; Tac antigen | 3559 | CD25 |
| CD26 | EC 3.4.14.5; ADA-binding protein; DPP; IV ectoenzyme | 1803 | CD26 |
| CD27 | S152; T14 | 939 | CD27 |
| CD28 | T44; Tp44 | 940 | CD28 |
| CD29 | Platelet GPIIa; VLA-beta chain; beta-1 integrin chain | 3688 | |
| CD30 | Ber-H2 antigen; Ki-1 antigen | 943 | CD30 |
| CD31 | GPiia'; endocam; PECAM-1 | 5175 | CD31 |
| CD32 | FCR II; Fc gamma RII | 2212 | |
| CD33 | gp67; p67 | 945 | CD33 |
| CD34 | gp105-120 | 947 | CD34 |
| CD35 | C3bR; C4bR; CR1; Immune Adherence Receptor | 1378 | CD35 |
| CD36 | GPIIIb; GPIV; OKM5-antigen; PASIV | 948 | CD36 |
| CD37 gp52-40 | | 951 | CD37 |
| CD38 | T10; cyclic ADP-ribose hydrolase | 952 | CD38 |
| CD39 | | 953 | |
| CD40 | Bp50 | 958 | CD40 |
| CD41 | GPIIb; alpha IIb integrin chain | 3674 | |
| CD42a | GPIX | 2815 | CD42a |
| CD42b | GPIbalpha; Glycocalicin | 2811 | CD42b |
| CD42c | GPIb-beta | 2812 | CD42c |
| CD42d | GPV | 2814 | CD42d |
| CD43 | gpL115; leukocyte sialoglycoprotein; leukosialin; sialophorin | 6693 | CD43 |
| CD44 | ECMR III; H-CAM; HUTCH-1; Hermes; Lu, In-related; Pgp-1; gp85 | 960 | CD44 |
| CD44R CD44v; | CD44v9 | 960 | CD44R |
| CD45 | B220; CD45R; CD45RA; CD45RB; CD45RC; CD45RO; EC 3.1.3.4; LCA; T200; Ly5 | 5788 | CD45 |
| CD46 | MCP | 4179 | CD46 |
| CD47R | Rh-associated protein; gp42; IAP; neurophilin; OA3; MEM-133; formerly CDw149 | 961 | CD47 |
| CD48 | BCM1; Blast-1; Hu Lym3; OX-45 | 962 | CD48 |
| CD49a | Alpha-1 integrin chain; VLA-1 alpha chain | 3672 | |
| CD49b | Alpha-2 integrin chain; GPIa; VLA-2 alpha chain | 3673 | |
| CD49C | alpha-3 integrin chain; VLA-3 alpha chain | 3675 | |
| CD49d | Alpha-4 integrin chain; VLA-4 alpha 3676 chain | | CD49d |
| CD49e | Alpha-5 integrin chain; FNR alpha chain; VLA-5 alpha chain chain; VLA-5 alpha chain | 3678 | |
| CD49f | Alpha-6 integrin chain; Platelet gpI; VLA-6 alpha chain | 3655 | |
| CD50 | ICAM-3 | 3385 | CD50 |
| CD51 | VNR-alpha chain; alpha V integrin chain; vitronectin receptor | 3685 | |
| CD52 | | 1043 | CD52 |
| CD53 | | 963 | CD53 |
| CD54 | ICAM-1 | 3383 | CD54 |
| CD55 | DAF | 1604 | CD55 |
| CD56 | Leu-19; NKH1; NCAM | 4684 | CD56 |
| ICD57 | HNK1; Leu-7 | 964 | |
| CD58 | LFA-3 | 955 | CD58 |
| CD59 | 1F-5Ag; H19; HRF20; MACIF; MIRL; P-18; Protectin | 966 | CD59 |
| CD60a | GD3 | | CDw60 |
| CD60b | 9-O-acetyl-GD3 | | CDw60 |
| CD60c | 7-O-acetyl-GD3 | | CDw60 |
| CD61 | CD61A; GPIIb/IIIa; beta 3 integrin chain | 3590 | |
| CD62E | E-selectin; ELAM-1; LECAM-2 | 6401 | CD62E |
| CD62L | L-selectin; LAM-1; LECAM-1; Leu-8; MEL-14; TQ-1 | 6402 | CD62L |
| CD62P | P-selectin; GMP-140; PADGEM | 6403 | CD62P |
| CD63 | LIMP; MLA1; PTLGP40; gp55; granulophysin; LAMP-3; ME491; NGA | 967 | CD63 |
| CD64 | FC gammaRI; FCR I | 2209 | CD64 |
| CD65 | Ceramide-dodecasaccharide; VIM-2 | | |
| CD65s | Sialylated-CD65; VIM2 | | |
| CD66a | NCA-160; BGP | 634 | CD66a |
| CD66b | CD67; CGM6; NCA-95 | 1088 | CD66b |
| CD66c | NCA; NCA-50/90 | 4680 | CD66c |
| CD66d | CGM1 | 1084 | CD66d |
| CD66e | CEA | 1048 | CD66e |
| CD66f | Pregnancy specific b1 glycoprotein; SP-1; PSG | 5669 | CD66f |
| CD68 | gp110; macrosialin | 968 | CD68 |
| CD69 | AIM; EA 1; MLR3; gp34/28; VEA | 969 | CD69 |
| CD70 | CD27-ligand; Ki-24 antigen | 970 | |
| CD71 | T9; transferrin receptor | 7037 | CD71 |
| CD72 | Ly-19.2; Ly-32.2; Lyb-2 | 971 | |
| CD73 | Ecto-5'-nucleotidase | 4907 | CD73 |
| CD74 | Class II-specific chaperone; Ii; Invariant chain | 972 | CD74 |
| CD75 | Lactosamines | | |
| CD75s | Alpha-2,6-sialylated lactosamines (formerly CDw75 and CDw76) | | CDw75; CDw76 |
| CD77 Pk | blood group antigen; BLA; CTH; Gb3 | | CD77 |
| CD79a | Ig alpha; MB1 | 973 | |
| CD79b | B29; Ig beta | 974 | |
| CD80 | B7; BB1 | 941 | CD80 |
| CD81 | TAPA-1 | 975 | CD81 |
| CD82 | 4F9;C33;IA4;KAI1; R2 | 3732 | CD82 |
| CD83 | HB15 | 9308 | CD83 |
| CD84 | | 8832 | CD84 |
| CD85 | ILT/LIR family {Young NT/Parham P.2001.IMMUN} {Allan DS/Braud VM.2000.IMMUN} | 10859 | CD85 |
| CD86 | B7-2;B70 | 942 | CD86 |
| CD87 | uPAR | 5329 | CD87 |
| CD88 | C5aR | 728 | CD88 |
| CD89 | Fcalpha-R; IgA Fc receptor; IgA receptor | 2204 | CD89 |
| CD90 | Thy-1 | 7070 | CD90 |
| CD91 | ALPHA2M-R; LRP | 4035 | |
| CD92 | CTL1; formerly CDw92 | 23446 | CD92 |
| CDw93 | | 23447 | CDw93 |
| CD94 | Kp43 | 3824 | CD94 |
| CD95 | APO-1; Fas; TNFRSF6; APT1 | 355 | CD95 |
| CD96 | TACTILE | 10225 | |
| CD97 | | 976 | CD97 |
| CD98 | 4F2; FRP-1; RL-388 | 4198 | CD98 |
| CD99 | CD99R; E2; MIC2 gene product | 4267 | |
| CD100 | SEMA4D | 10507 | CD100 |
| CD101 | IGSF2; P126; V7 | 9398 | CD101 |
| CD102 | ICAM-2 | 3384 | CD102 |
| CD103 | ITGAE; HML-1; integrin alphaE chain | 3682 | CD103 |
| CD104 | beta 4 integrin chain; TSP-1180; beta 4 | 3691 | |
| CD105 | endoglin | 2022 | CD105 |
| CD106 | INCAM-110; VCAM-1 | 7412 | |
| CD107a | LAMP-1 | 3916 | CD107a |
| CD107b | LAMP-2 | 3920 | CD107b |
| CD108 | SEMA7A; JMH human blood group antigen; formerly CDw108 antigen; formerly CDw108 | 8482 | CD108 |
| CD109 | 8A3; E123; 7D1 | | |
| CD110 | MPL; TPO-R; C-MPL | 4352 | |
| CD111 | PVRL1; PRR1; HevC; nectin-1; HIgR | 5818 | |
| CD112 | HVEB; PRR2; PVRL2; nectin 2 | 5819 | |
| CD113 | Reserved | | |
| CD114 | CSF3R; HG-CSFR; G-CSFR | 1441 | CD114 |
| CD115 | c-fms; CSF-1R; M-CSFR | 1436 | |
| CD116 | GM-CSF receptor alpha chain | 1438 | CD116 |
| CD117 | c-KIT; SCFR | 3815 | CD117 |
| CD118 | Reserved | | |
| CDw119 | IFNgR; IFNgRa | 3459 | |
| CD120a | TNFRI; p55 | 7132 | |
| CD120b | TNFRII; p75; TNFR p80 | 7133 | |
| CD121a | IL-1R; type 1 IL-1R | 3554 | |
| CDw121b | IL-1R, type 2 | 7850 | |
| CD122 | IL-2Rbeta | 3560 | CD122 |
| CD123 | IL-3Ralpha | 3563 | |
| CD124 | IL-4R | 3566 | CD124 |
| CDw125 | IL-5Ralpha | 3568 | CDw125 |
| CD126 | IL-6R | 3570 | CD126 |
| CD127 | IL-7R; IL-7R alpha; p90 I17 R | 3575 | CD127 |
| CDw128a | CXCR1; IL-8RA | 3577 | |
| CDw128b | CXCR2; IL-8RB | 3579 | |
| CD129 | Reserved | | |
| CD130 | gp130 | 3572 | CD130 |
| CD131 | common beta subunit | 1439 | CDw131 |
| CD132 | IL2RG; common cytokine receptor gamma chain; common gamma chain | 3561 | CD132 |
| CD133 | PROML1; AC133; hematopoietic stem cell antigen; prominin-like 1 | 8842 | |
| CD134 | OX40 | 7293 | |
| CD135 | flt3; Flk-2; STK-1 | 2322 | CD135 |
| CDw136 | msp receptor; ron; p158-ron | 4486 | CDw136 |
| CDw137 | 4-1BB; ILA | 3604 | CDw137 |
| CD138 | heparan sulfate proteoglycan; syndecan-1 | 6382 | |
| CD139 | | 23448 | CD139 |
| CD140a | PDGF-R; PDGFRa 5156 | | |
| CD140b | IPDGFRb | 5159 | |
| CD141 | fetomodulin; TM | 7056 | CD141 |
| CD142 | F3; coagulation Factor III; thromboplastin; TF | 2152 | CD142 |
| CD143 | EC 3.4.15.1; ACE; kininase II; peptidyl dipeptidase A | 1636 | CD143 |
| CD144 | cadherin-5; VE-Cadherin | 1003 | CD144 |
| CDw145 | | | |
| CD146 | MCAM; A32; MUC18; Mel-CAM; S-endo | 4162 | CD146 |
| CD147 | 5A11; Basigin; CE9; HT7; M6; Neurothelin; OX-47; EMMPRIN; gp42 | 682 | CD147 |
| CD148 | HPTP-eta; DEP-1; p260 | 5795 | CD148 |
| CDw149 | new designation is CD47R | | |
| CD150 | SLAM; IPO-3; fomerly CDw150 | 6504 | CDw150 |
| CD151 | PETA-3; SFA-1 | 977 | CD151 |
| CD152 | CTLA-4 | 1493 | CD152 |
| CD153 | CD30L | 944 | |
| CD154 | CD40L; T-BAM; TRAP; gp39 | 959 | |
| CD155 | PVR | 5817 | |
| CD156a | ADAM8; MS2 human; fomerly CD156 | 101 | CD156a |
| CD156b | ADAM17; TACE; cSVP | 6868 | |
| CD157 | BP-3/IF-7; BST-1; Mo5 | 683 | CD157 |
| CD158 | KIR family (detailed nomenclature to be published) | | KIR Family |
| CD159a | NKG2A | 3821 | |
| CD160 | BY55 antigen; NK1; NK28 | 111126 | |
| CD161 | KLRB1; NKR-P1A; killer cell lectin-like receptor subfamily B, member 1 | 3820 | CD161 |
| CD162 | PSGL-1, PSGL | 6404 | CD162 |
| CD162R | PEN5 (a post-translational modification of PSGL-1) | 6404 | |
| CD163 | GHI/61; M130; RM3/1 | 9332 | |
| CD164 MUC-24; | MGC-24v | 8763 | |
| CD165 | AD2; gp37 | 23449 | CD165 |
| CD166 | BEN; DM-GRASP; KG-CAM; Neurolin; SC-1; ALCAM | 214 | CD166 |
| CD167a | trkE; trk6; cak; eddr1; DDR1; MCK10; RTK6; NTRK4 | 780 | |
| CD168 | HMMR; IHABP; RHAMM | 3161 | |
| CD169 | sialoadhesin; siglec-1 | 6614 | |
| CD170 | Siglec-5 | 8778 | |
| CD171 | L1; L1CAM; N-CAM L1 | 3897 | |
| CD172a | SIRP alpha | 8194 | |
| CD173 | Blood group H type 2 | | |
| CD174 | Lewis y | 2525 | |
| CD175 | Tn | | |
| CD175s Sialyl-Tn | | | |
| CD176 | TF | | |
| CD177 | NB1 | | |
| CD178 | fas-L; TNFSF6; APT1LG1; CD95-L | 356 | |
| CD179a | VpreB; VPREB1; IGVPB | 7441 | |
| CD179b | IGLL1; lambda5; immunoglobulin omega polypeptide; IGVPB; 14.1 chain | 3543 | |
| CD180 | LY64; RP105 | 4064 | |
| CD183 | CXCR3; GPR9; CKR-L2; IP10-R; Mig-R | 2833 | |
| CD184 | CXCR4; fusin; LESTR; NPY3R; HM89; FB22 | 7852 | |
| CD195 | CCR5 | 1234 | |
| CDw197 | CCR7 | 1236 | |
| CD200 | OX2 | 4345 | |
| CD201 | EPC R | 10544 | |
| CD202b | tie2; tek | 7010 | |
| CD203c | NPP3; PDNP3; PD-Ibeta; B10; gp130RB13-6; ENPP3; bovine intestinal phosphodiesterase | 5169 | |
| CD204 | macrophage scavenger R | 4481 | |
| CD205 | DEC205 | 4065 | |
| CD206 | MRC1; MMR | 4360 | |
| CD207 | Langerin | 50489 | |
| CD208 | DC-LAMP | 27074 | |
| CD209 | DC-SIGN | 30385 | |
| CDw210 | IL-10 R | 3587; 3588 | |
| CD212 | IL-12 R | 3594 | |
| CD213a1 | IL-13 R alpha 1 | 3597 | |
| CD213a2 | IL-13 R alpha 2 | 3598 | |
| CDw217 | IL-17 R | 23765 | |
| CD220 | Insulin R | 3643 | |
| CD221 | IGF1 R | 3480 | |
| CD222 | Mannose-6-phosphate/IGF2 R | 3482 | |
| CD223 | LAG-3 | 3902 | |
| CD224 | GGT; EC2.3.2.2 | 2678 | |
| CD225 | Leu13 | 8519 | |
| CD226 | DNAM-1; PTA1; TLiSA1 | 10666 | |
| CD227 | MUC1; episialin; PUM; PEM; EMA; DF3 antigen; H23 antigen | 4582 | |
| CD228 | melanotransferrin | 4241 | |
| CD229 | Ly9 | 4063 | |
| CD230 | Prion protein | 5621 | |
| CD231 | TM4SF2; A15; TALLA-1; MXS1; CCG-B7; TALLA | 7102 | |
| CD232 | VESP R | 10154 | |
| CD233 | band 3; erythrocyte membrane protein band 3; AE1; SLC4A1; Diego blood group; EPB3 | 6521 | |
| CD234 | Fy-glycoprotein; Duffy antigen | 2532 | |
| CD235a | Glycophorin A | 2993 | |
| CD235b | Glycophorin B | 2994 | |
| CD235ab | Glycophorin A/B crossreactive mabs | | |
| CD236 | Glycophorin C/D | | |
| CD236R | Glycophorin C | 2995 | |
| CD238 | Kell | 3792 | |
| CD239 | B-CAM | 4059 | |
| CD240CE | Rh30CE | 6006 | |
| CD240D | Rh30D | 6007 | |
| CD240DCE | Rh30D/CE crossreactive mabs | | |
| CD241 | RhAg | 6005 | |
| CD242 | ICAM-4 | 3386 | |
| CD243 | MDR-1 | 5243 | |
| CD244 | 2B4; NAIL; p38 | 51744 | |
| CD245 | p220/240 | | |
| CD246 | Anaplastic lymphoma kinase | 238 | |
| CD247 | Zeta chain | 919 | |

Revised 07/02/02 prow@ncbi.nlm.nih.gov

The expression "the group of CD1 to CD247" as used herein means all the CD molecules in the list above.

In the most preferred embodiment, the anti-lymphoma antibody is directed against CD19, CD20, CD22, CD 30, in particular CD 20.

In the present patent application, an immunotargeting agent (immunoconjugate) is an agent carrying a cytotoxic moiety that, contrary to common cytotoxic medical agents, binds specifically to lymphatic tumor cell with a high affinity and which could be administered parentally, preferably intravenously, to a human being. In a preferred application, the immunotargeting agents are antibodies, which could be of different isotypes and could originate from any species. Of particular interest are the monoclonal antibodies and derivatives thereof. The latter include fragments such as the F(ab')₂, F(ab'), F(ab) and the like. They also include genetically engineered

hybrids or chemically synthesized peptides based on the specificity of the antigen binding region of one or several target specific monoclonal antibodies, e.g. chimeric or humanized antibodies, single chain antibodies etc.

The biomolecule binding moiety, which is an anti-lymphoma antibody reactive moiety, is bound or conjugated to the anti-lymphoma antibody, either covalently or non-covalently with an affinity binding constant of at least 10⁸M⁻¹.

The term "anti-lymphoma antibody" used herein is intended to mean an antibody with the ability of specific binding to a CD antigen on lymphoma tumour cells with an affinity binding constant of at least 5x10⁶M⁻¹, preferably at least 10⁸M⁻¹.

The term "variants" of the anti-lymphoma antibody as used herein means any modifications, fragments or derivatives thereof having the same or esentially similar affinity binding constant when binding to the CD antigen molecule, i.e. an affinity binding constant of at least 5x10⁶M⁻¹, preferably at least 10⁸M⁻¹.

Any of these variants could have been modified by the coupling of various number of polyethylene glycol chains in order to optimise the half-life in body fluid and the retention of the antibody or antibody fragments or derivatives, in the tumor tissue. In the most preferred application, the antibodies or antibody derivatives should allow for the attachment of a sufficient number of biotin residues to be used for extracorporeal removal through interaction with immobilized avidin, without significantly diminishing the binding properties of the targeting agent.

In order to enhance the specificity, tumour specific monoclonal antibodies are used as a carrier (immunoconjugates) of various cytotoxic moieties, such as, but not limited to, radio nuclides, chemotherapy drugs, synthetic or natural occurring toxins, immunosuppressive agents, immunostimulating agents and enzymes used in pro-drug protocols. The cytotoxic moiety is preferably a radionuclide such as a gamma-emitter e.g. iodine-131 or metal ion conjugate, where the metal is selected from a beta-particle emitter, such as yttrium or rhenium. US Patent No. 4,472,509, Gansow, et al., discloses the use of diethylenetriaminepentaacetic acid (DTPA) chelating agents for the binding of radio metals to monoclonal antibodies. The patent is particularly directed to a purification technique for the removal of non-bonded and adventitious-ly bonded (non-chelated) metal from radiopharmaceuticals but is illustrative of art recognized protocols for preparation of radionuclide labelled antibodies.

According to such general procedures, an antibody specifically reactive with the target tissue associated antigen is reacted with a certain quantity of a selected bifunctional chelating agent having protein binding and metal binding functionalities to produce a chelator/- antibody conjugate. In conjugating the antibodies with the chelators, an excess of chelating agent is reacted with the antibodies, the specific ratio being dependent upon the nature of the reagents and the desired number of chelating agents per antibody. It is a requirement that the radionuclides be bound by chelation (for metals) or covalent bonds in such a manner that they do not become separated from the biotinylation/radiolabeling compound under the conditions that the biomolecule conjugates is used (e.g. in patients). Thus, the most stable chelates or covalent bonding arrangements are preferred. Examples of such binding/bonding moieties are: aryl halides and vinyl halides for radionuclides of halogens; N₂S₂ and N₃S chelates for Tc and Re radionuclides; amino-carboxy derivatives such as EDTA , DTPA, derivatives of Me-DTPA and Cyclohexyl-DTPA, and cyclic amines such as NOTA, DOTA, TETA, CITC-DTPA, and triethylenetetraaminehexaacetic acid derivatives (Yuangfang and Chuanchu, Pure & Appl. Chem. 63, 427-463, 1991) for In, Y, Pb, Bi, Cu, Sm, and Lu radionuclides, and where the radionuclide is, but not limited to, any of the following elements:
Beta radiation emitters, which are useful as cytotoxic agents, include isotopes such as scandium-46, scandium-47, scandium-48, copper-67, gallium-72, gallium-73, yttrium-90, ruthenium-97, palladium-100, rhodium-101, palladium-109, samarium-153, lutetium-177, rhenium-186, rhenium-188, rhenium-189, gold-198, radium-212 and lead-212. The most useful gamma emitters are iodine-131 and indium-m114. Other metal ions useful with the invention include alpha radiation emitting materials such as 212-bismuth, 213-bismuth, and At-211 as well as positron emitters such as gallium-68 and zirconium-89.

In another embodiment of the invention, radionuclide-labelled targeting agents are useful not only in the treatment of lymphatic cancers, but also for imaging of such cancers.

At a suitable time after administration, " cytotoxic targeting agents" will be cleared from the blood system by extracorporeal means. To facilitate the extracorporeal depletion, an apparatus for extracorporeal circulation of whole blood or plasma will be connected to the patient through tubing lines and blood access device (s). Such an apparatus should provide conduits for transporting the blood to an adsorption device and conduits for returning the processed blood or plasma to the patient. In the case plasma is processed through the adsorption device, a plasma separation device is needed as well as means of mixing the concentrated blood with processed plasma. The latter is normally achieved by leading the two components into an air-trap where the mixing occurs.

In the case where whole blood is processed, an ordinary dialysis machine can constitute the base for such an apparatus. Dialysis machines are normally equipped with all the necessary safeguards and monitoring devices to meet patient safety requirements as well as to allow easy handling of the system. Hence, in a preferred embodiment whole blood is processed and a standard dialysis machine is utilised with only minor modifications of the hardware. However, such a machine requires a new program fitted to the newly intended purpose.

In addition to the apparatus, special blood line tubings suitable for the intended flow and distance from the patient and the machine are needed. These line tubings could be made of any material compatible with blood or plasma and would include materials used in ordinary tubings used in dialysis.

Blood access could be achieved through peripheral vein catheters or, if higher blood flow is needed, through central vein catheters such as, but not limited to, subclavian or femoral catheters.

For affinity adsorbents, the matrix may be of various shape and chemical composition. It may, for example, constitute a column house filled with particulate polymers, the latter of natural origin or artificially made. The particles may be macroporous or their surface may be grafted, the latter in order to enlarge the surface area. The particles may be spherical or granulated and be based on polysaccharides, ceramic material, glass, silica, plastic, or any combination of these or alike materials. A combination of these could, for example, be solid particles coated with a suitable polymer of natural origin or artificially made. Artificial membranes may also be used. These may be flat sheet membranes made of cellulose, polyamide, polysulfone, polypropylene or other types of material which are sufficiently inert, biocompatible, nontoxic and to which the receptor could be immobilized either directly or after chemical modification of the membrane surface. Capillary membranes like the hollow fibers made from cellulose, polypropylene or other materials suitable for this type of membranes may also be used. A preferred embodiment is a particulate material based on agarose and suitable for extracorporeal applications.

In one embodiment, an affinity label is attached to the anti-lymphoma antibody and the adsorption device contains an immobilized receptor binding specifically to the affinity ligand. Any type of affinity ligand/immobilized receptor combinations such as "antibodies and antigens/haptens" and "protein and co-factors" could be used in the this application, provided that they exhibit a sufficiently high binding affinity and selectively to the tumor markers, and that the affinity ligand-receptor interaction is not interfered with by blood or other body fluids or tissues being in contact with the immunotargeting agent and/or the device.

In one of the most preferred applications, the affinity ligand/immobilized receptor combination is biotin or biotin derivatives and biotin binding molecules, in particular where the affinity ligand is biotin or derivatives thereof and the immobilized receptor is avidin or streptavidin or any other biotin binding molecule. The affinity ligand pairs of biotin/avidin and biotin/streptavidin are often used with biomolecules. The very strong interaction (i.e. K = 10¹³ - 10¹⁵ M⁻¹) of biotin with the proteins avidin and streptavidin (Green, Methods Enzymol. 184, 51-67, 1990; Green, Adv. Prot. Chem. 29, 85-133, 1975) provides a foundation for their use in a large number of applications, both for *in vitro* and *in vivo* uses. A further application of the invention is the simultaneous removal of several different biotinylated "anti-cancer agents" through the same extracorporeal procedure.

The reagent used in the present invention is schematically shown below, wherein the biomolecule reactive moiety is an anti-lymphoma reactive moiety.

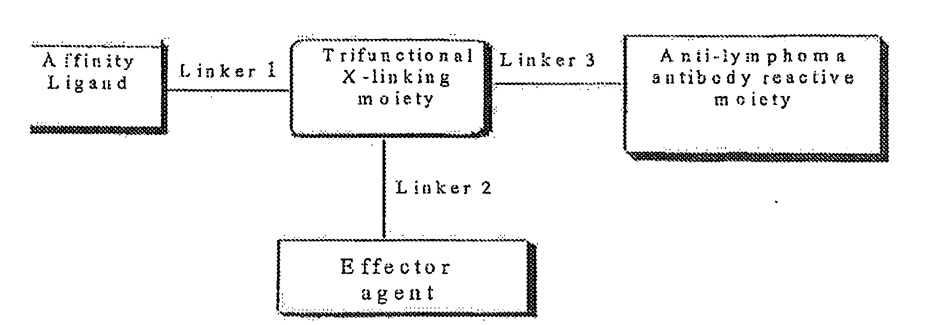

The medical agent according to the present invention is schematically shown below, wherein an anti-lymphoma antibody is bound or conjugated to the reagent via the anti-lymphoma antibody reactive moiety of the reagent.

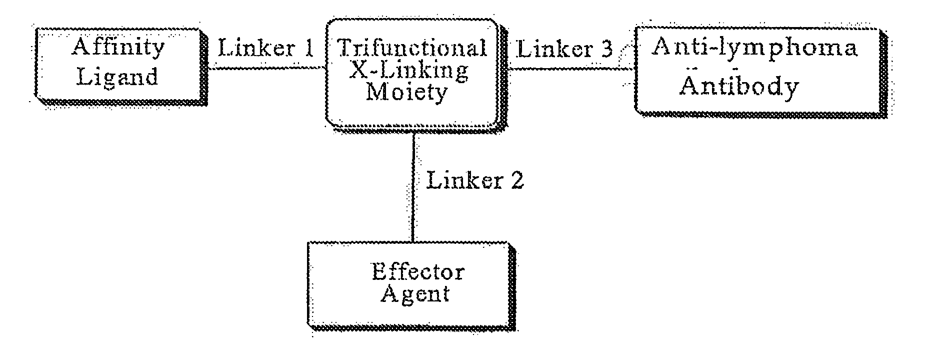

In the schematically shown reagent and medical agent, respectively, different which components will be presented in more detail below.

The anti-lymphoma antibody reactive moiety is chosen from a group of active esters consisting of N-hydroxysuccinimide esters, sulfo-N-hydroxysuccinimide esters, and phenolic esters; aryl and alkyl imitates; alkyl or aryl isocyanates or isothiocyanates reacting with amino groups on the anti-lymphoma antibody, or maleimides or alpha-haloamides reacting with sulfhydryl groups on the anti-lymphoma antibody; or aryl or alkylhydrazines or alkyl or arylhydroxylamines reacting with aldehyde or ketone groups naturally occurring or synthetically produced on the anti-lymphoma antibody, or variants thereof.

The effector agent is a radionuclide binding moiety, optionally provided with a radionuclide, a synthetic or naturally occurring toxin, an enzyme capable of converting pro-drugs to active drugs, immunosuppressive or immunostimulating agents, radiosensitizers, enhancers for X-ray or MRI or ultrasound, non-radioactive elements, which can be converted to radio active elements by means of external irradiation after that the anti-lymphoma antibody carrying said element has been accumulated to specific cells or tissues, or photoactive compounds or compounds used in photo imaging or photo dynamic therapy, or any other molecule having the same or similar effect, directly or indirectly, on lymphoma cells or lymphoma tissues. More precisely, the effector agent comprises Me-DTPA, CITC-DTPA, and cyclohexyl-DTPA.

The affinity ligand can be any moiety that binds with another molecule with an affinity constant of 10⁶ M⁻¹ or higher. A preferred affinity ligand is a moiety which binds specifically to avidin, streptavidin, or any other derivatives, mutants or fragments of avidin or streptavidin having essentially the same binding function to the affinity ligand. Preferably, the affinity ligand is biotin, or a biotin derivative having essentially the same binding function to avidin or streptavidin as biotin. Said biotin derivative may be chosen from the group consisting of a biotin derivative having essentially the same binding function to avidin or streptavidin as biotin.

The anti-lymphoma antibody having ability to be conjugated to said anti-lymphoma antibody reactive moiety interacts with one or more different cell surface antigen(s) present on the surface of lymphoma tumour cells, said one or more cell surface antigen(s) being one or more different CD antigen(s), or variants thereof, wherein the anti-lymphoma antibody preferably is chosen from anti-CD20 antibodies, preferably rituximab, ibritumomab and tositumomab.

The trifunctional cross-linking moiety is chosen from the group consisting of triaminobenzene, tricarboxy-benzene, dicarboxyanyline and diaminobenzoic acid.

Linker 1 is a chemical moiety that is an attaching moiety and spacer between the trifunctional cross-linking moiety and the affinity ligand, preferably a biotin moiety, such that binding with avidin or streptavidin, or any other biotin binding species, is not diminished by steric hindrance. Linker 1 may also impart increased water solubility and biotinidase stabilization, preferably against cleavage by biotinidase by introduction of an alpha carboxylate or an N-methyl group. Further, it contains hydrogen bonding atoms, preferably ethers or tioethers, or ionisable groups, preferably carboxylate, sulfonates, or ammonium groups to aid in water solubilisation of the biotin moiety.

For the structural requirements of the biotin containing moiety, the following applies with reference to the following embodiment of the present invention:
generalized structure of a 1033-anti-CD20 antibody

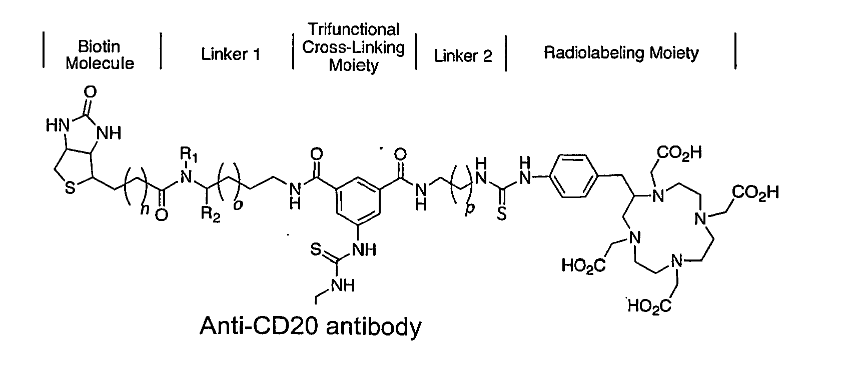

This structure is bound to the effector agent, wherein the anti-CD20 antibody preferably is rituximab, wherein n is 2-4, preferably 3; o is 1-6, preferably 3; p is 1-6, preferably 3; R₂ is =CH₂OH or -CO₂H; and R₁ is - CH₃, -CH₂OH, or -H.

There are three aspects of the biotin portion of the 1033 structures that are important in this application:
(1) blockage of biotinidase cleavage, (2) retention of high biotin binding affinity, and (3) attainment of a reasonable aqueous solubility. To provide these attributes, biotin conjugates must be composed of a biotin molecule and an appropriate linker, which are coupled to a cross-linking moiety.

Biotin conjugates must be prepared by conjugation with the carboxylate on the pentanoic acid side chain (n = 3). Conjugation at other locations in the biotin molecule results in complete loss of binding with avidin and streptavidin. This renders the biotin molecule useless for this application. The preferred form of conjugation is formation of an amide bond with the carboxylate group (as depicted in the general formula). Since binding of biotin with avidin and streptavidin is in a deep pocket (e.g. 9Å), shortening (n<3) or lengthening (n>3) of the pentanoic acid side chain results in low binding affinity, which is not desired for this application.

Blocking of biotinidase activity is achieved by attaching appropriate substituents to the biotinamide amine (i.e. R₁) or to an atom adjacent to that amine (i.e. R₂). Biotinidase is an enzyme that cleaves (hydrolyzes) the amide bond of biotin carboxylate conjugates. This enzyme is very important in recycling biotin in animals and man. Metabolism of biotin in (several different) protein carboxylases releases biotin-w-N-lysine (biocytin), and biotinidase specifically cleaves that amide bond to release free biotin. Biotinidase is also capable of cleaving (non-specifically) other biotinamide bonds. In this application, it is important that biotinidase do not cleave biotin from the conjugates, otherwise the desired outcome will not be achieved. Thus, the useful biotin conjugate structures incorporate functional groups (R₁ or R₂) that block the enzymatic activity of biotinidase. While it is likely that any structure for R₁ will block biotinidase, its structure is generally limited to a methyl (CH₃) group, as this group completely blocks biotinidase activity. The N-methyl group decreases the binding affinity of biotin with avidin and streptavidin significantly, but it is still useful in this application. Larger groups for R₁ (e.g. ethyl, aryl, etc.) are not useful due to the loss of binding affinity. The alternative to having a substituent R₁ is to have a substituent R₂ on the atom (e.g. methylene) adjacent to the biotinamide amine. Much larger and more varied substituents can be used in this position without any significant effect on the binding affinity of biotin. Biotinidase is not completely blocked when R₂ = CH₃ or CH₂CH₃, although the rate of cleavage is slowed considerably (i.e. to 25% and 10% respectively). Complete blockage of biotinidase activity is attained when R₂ = CH₂OH and CO₂H functionalities. The important consideration is that there is no decrease in binding affinity when these groups are incorporated as R₂. Larger functional groups can also be used as R₂ to block biotinidase activity, but results in a decrease in binding affinity. The larger functional groups as R₂ are useful in this application if they do not cause a decrease in binding affinity greater than that obtained when R₁ = CH₃.

The biotin affinity and water solubility of the biotin moiety in 1033 are affected by the linker moiety used. The length and nature of the linker moiety (Linker 1) will be dependent to some degree on the nature of the molecule that it is conjugated with. The linker moiety serves the function of providing a spacer between the biotin moiety and the rest of the conjugate such that the biotin binding is not affected by steric hindrance from the protein (or other conjugated molecule). The length (number of atoms in a linear chain) of the linker may vary from o = 4-20 for conjugates with small molecules (e.g. steroids) to o > 20 for large conjugate molecules (e.g. IgG molecule). The nature of the atoms in the linker (linear chain or branch from it) will also vary to increase water solubility. For example, linkers that contain more than 4 methylene units are improved by incorporation of oxygen or sulfur atoms (forming ethers or thioethers) or by having appended ionizable functionalities (e.g. sulfonates, carboxylates, amines or ammonium groups).

Linker 2, if present, is a chemical moiety that is used to attach the radionuclide binding moiety to the trifunctional cross-linking moiety. It provides a spacer length of 1-25 atoms, preferably a length of 6-18 atoms, or groups of atoms. Linker 2 may also impart increased water solubility due to the presence of hydrogen bonding atoms, preferably esters or bioeters, or ionisable groups, to aid in water solubilisation.

Linker 3 may not be required, but where advantageous, it is a chemical moiety used to attach the biomolecule reactive moiety to the trifunctional cross-linking moiety. Linker 3 may be used as a spacer with a length of 1-25 atoms, preferably 6-18 atoms, or groups of atoms and/or it may be used to increase the water solubility of the compound due to the presence of hydrogen bonding atoms, such as ethers or tioethers, or ionisable groups, preferably carboxylate, sulfonates, or ammonium groups to aid in water solubilisation.

Moreover, the reagent according to the present invention may contain more than one affinity ligand and/or more than one effector agent bound to a trifunctional or tetrafunctional cross-linking moiety.

One embodiment of the medical agent has the following schematic structure : 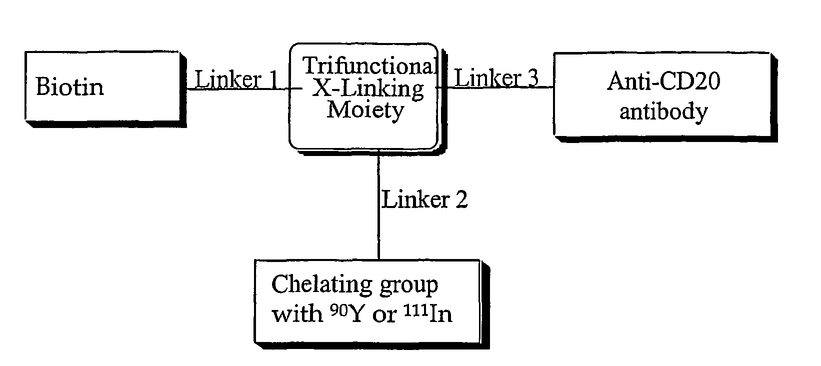 where the chelating group is, but not limited to, any of the following compounds: aryl halides and vinyl halides for radionuclides of halogens; N₂S₂ and N₃S chelates for Tc and Re radionuclides; amino-carboxy derivatives such as EDTA, DTPA, derivatives Me-DTPA and Cyclohexyl-DTPA , and cyclic amines such as NOTA, DOTA, TETA, CITC-DTPA, and triethylenetetraaminehexaacetic acid derivatives (Yuangfang and Chuanchu, Pure & Appl. Chem. 63, 427-463, 1991) for In, Y, Pb, Bi, Cu, Sm, Lu radionuclides and where the radionuclide is, but not limited, any of the following elements: Beta radiation emitters, which are useful as cytotoxic agents, include isotopes such as scandium-46, scandium-47, scandium-48, copper-67, gallium-72, gallium-73, yttrium-90, ruthenium-97, palladium-100, rhodium-101, palladium-109, samarium-153, lutetium-177, rhenium-186, rhenium-188, rhenium-189, gold-198, radium-212 and 212 lead. The most useful gamma emitters are 1iodine-131 and indium-m114. Other metal ions useful with the invention include alpha radiation emitting materials such as 212-bismuth, 213-bismuth, and At-211 as well as positron emitters such as gallium-68 and zirconium-89.

In the most preferred embodiment of the present invention, the medical agent is the rituximab conjugate with 1-5 groups of 3-(13'-ThioureabenzylDOTA)Trioxadi-amine-1-(13"-Biotin-Asp-OH)Trioxadiamine-5-isothiocya-nato-Aminoisophthalate (see below). The radionuclide is ⁹⁰Y for therapeutic application and ¹¹¹In for in vivo diagnostic application. In the very most preferred embodiment, the rituximab conjugate contains 1.5 - 3.5 groups of 3-(13'-ThioureabenzylDOTA)Trioxadiamine-1-(13"-Biotin-Asp-OH)Trioxadiamine-5-isothiocyanato-Aminoisophthalate.

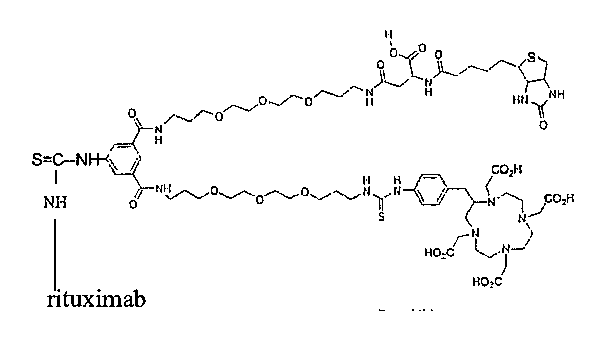

Ibritumomab or tositumomab is also effective as anti-lymphoma antibody in the medical agent.

### Examples

The following examples shall not be construed as limiting the invention, but should be regarded as evidence of the applicability of the invention.

### Example 1 - Conjugation and radiolabelling of rituximab.

In this and subsequent examples, Indium-111 has in some instances been used as a substitute for Yttrium-90, because the former is a gamma-emitter and possesses less radiation hazard than Yttrium-90.

The monoclonal antibody, Rituximab was conjugated with 3-(13'-ThioureabenzylDOTA)Trioxadiamine-1-(13"-Biotin-Asp-OH) trioxadiamine-5-Isothiocyanato-Aminoisophtalate (MitraTag-1033), for short also called "1033" in the following, using the method described by Wilbur D.S et al in Bioconjugate Chem. 13:1079-1092, 2002. A 5 mg quantity of the monoclonal antibody was dialysed against 1L metal free HEPES with a minimum of 5 buffer changes over 3 days at 4°C. A solution of MitraTag-1033 was made in water, and an appropriate volume was added to the antibody solution. After incubation overnight at room temperature, the antibody-conjugate was dialysed against 1L metal free 500 mM ammonium acetate buffer pH 5.3 with a minimum of 3 buffer changes over 3 days at 4°C. The demetalated conjugated antibody was stored at 4-8°C until used in radiolabelling experiments.

275 µl antibody conjugate (1375 µg; 1033-Rituximab) in 500 mM ammonium acetate buffer pH 5.3 was mixed with 15 µl ¹¹¹InCl₃ (or ⁹⁰YCl₃) in 50 mM HCl. The labelling was conducted at 45°C for 16 minutes. 28 µl DTPA was added to stop reaction. The quality of the radio conjugate was determined by TLC and HPLC. The number of MitraTag-1033 per monoclonal antibody molecule was determined by the HABA method.

### Example 2 - Binding of the 1033-conjugated monoclonal antibody to an avidin-adsorbent.

The fraction of the 1033-rituximab radio conjugate binding to the Avidin-adsorbent utilised in the Mitradep^{®} device, was analysed utilising micro-columns.

The non-bound protein fraction of a 2.4 conjugates/- IgG 1033-rituximab was 9%, and of a 4.6 conjugates/IgG 1033-rituximab 3%. This is well in line with a Poisson distribution of the conjugates. Hence, the above Rituximab conjugates should contain fractions, which are not labelled with MitraTag-1033. Hence, the non-binding fraction complies with the expected fraction of non-conjugated Rituximab i.e. the non-radioactive fraction.

More than 99% of the radioactivity in a radiolabelled 1033-conjugate sample was bound to the micro-column with the Avidin-adsorbent.

### Example 3 - Depletion of 1033-rituximab conjugates during in vitro simulated treatments.

The depletion kinetics of 1033-rituximab during a patient treatment was simulated *in vitro* utilising a recirculation method based on the principles described by Schindhelm K. (Artificial Organs 13:21-27 (1989)).

The 1033-rituximab was diluted in a solution with the same relative viscosity as human blood, and was re-circulated *in vitro* through a small-scale model of the Mitradep^{®} device. 125 ml of a blood substitute, containing 10 mg of 1033-rituximab, were re-circulated at 6.25 ml/min (corresponds to 100 ml/min in Mitradep^{®}). Three reservoir volumes were processed. The levels of 1033-rituximab in the reservoir were monitored.

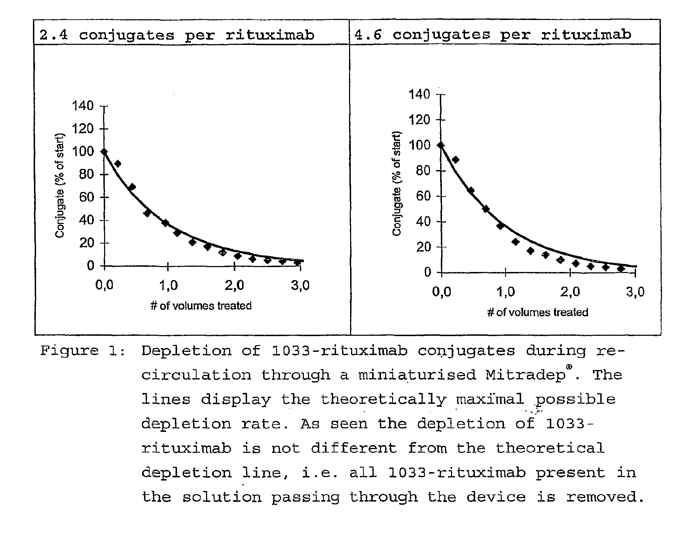

When two preparations of 1033-rituximab with different numbers of MitraTag^{™}-1033 moieties per Rituximab molecule were analysed, the results presented in Fig. 1 were obtained. As seen, the depletion of 1033-rituximab is not different from the theoretical depletion line, i.e. all 1033-rituximab present in the solution passing through the device is removed. Studies with biotinylated human IgG have shown that an efficient depletion is obtained at a biotin/IgG ratio down to 1.4 biotin/IgG (lowest ratio tested).

It was concluded that 1033-rituximab could be efficiently removed during an extracorporeal affinity adsorption procedure utilising the device Mitradep^{®}.

### Example 4 - Binding of the 1033-conjugate to the target antigen CD20.

After conjugation with MitraTag^{™}-1033, the 1033-rituximab conjugates were analysed for binding to the target antigen CD20 to confirm that the conjugation process has not denaturated the antigen binding. The CD20 antigen is not available in purified and soluble form. Therefore, during testing the CD20 expressing B-cell lymphoma cell lines, Raji and/or SB, was utilised as targets.

The specificity of the antigen binding was analysed by immunofluoroscence in a flowcytometry (FACS) method. Briefly, the Raji cells were incubated with biotinylated-Rituximab and 1033-rituximab conjugates. After incubation, the cells were washed and incubated with fluorescence-labelled Avidin. After washing the cells were analysed in the FACS instrument. As positive control, a biotinylated mouse monoclonal antibody against CD20 was used, as negative control a PBS buffer was used. For control of binding to F_{c}-receptors on the cells, biotinylated normal human IgG was used. The results are presented in graphs where the x-axis presents the amount of fluorescence per cell on a logarithmic scale, and the Y-axis the number of cells displaying the specified fluorescence.

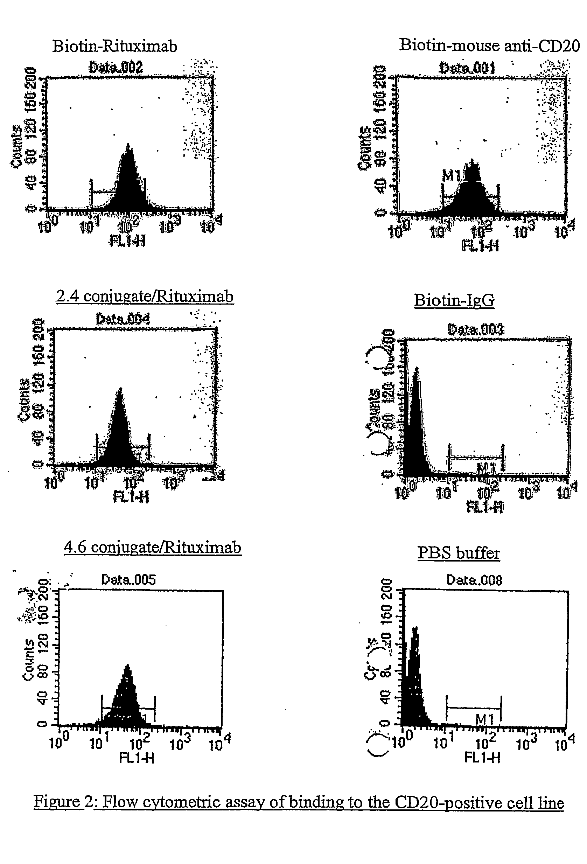

As seen in Fig. 2, no non-specific binding of Avidin to the cells was detected. Neither was binding to F_{c}-receptors seen utilising biotinylated human IgG. There was no significant difference in binding between the control mouse antibody, biotinylated Rituximab, or the two MitraTag^{™}-1033 conjugates tested.

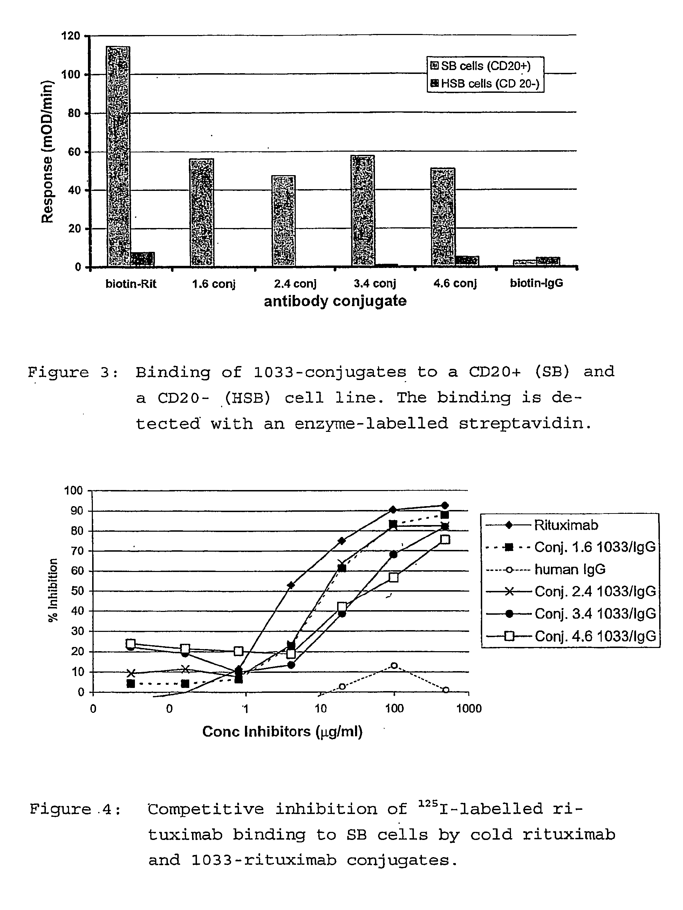

The specificity was also determined by analysing the binding of the conjugates to a CD20-positive cell line (SB) and a CD20-negative cell line (HSB) established from the same individual in an ELISA. The cells were dried into the wells of an ELISA plate. After incubation with 1033-rituximab conjugates, the bound antibodies were detected with an enzyme-conjugated Streptavidin. Biotinylated Rituximab and biotinylated normal human IgG were used as positive and negative control, respectively. As seen in Fig. 3, non-specific binding to the control cells was insignificant.

It was concluded that Rituximab retains the binding specificity to the antigen CD20 after conjugation with the MitraTag^{™}-1033 reagent.

### Example 5 - Analyses of the affinity of the binding to the CD20 antigen

The influence of the conjugation process on the binding affinity (strength) of Rituximab to the target antigen CD20 was studied utilising a competitive inhibition assay.

Briefly, increasing amounts of non-radiolabelled Rituximab and 1033-rituximab conjugates were mixed with a constant amount of ¹²⁵I-labelled Rituximab labelled utilising the Bolton-Hunter reagent. The mixtures were added to fixed SB lymphoma cells in 96 plate wells. After incubation for 2 hours at room temperature, the wells were washed, and the radioactivity bound to the cells was measured in an automatic NaI(Tl) scintillation well counter.

For each concentration of cold Rituximab and 1033-rituximab conjugates, the per cent inhibition of cell binding radioactivity was calculated. The per cent inhibition was plotted against concentration (Fig. 4), and the concentration required for 50% inhibition (IC₅₀) was calculated from the graph (Table 1). The IC₅₀ is a measure of the relative affinity (avidity) of the tested antibody; a decrease of affinity is seen as an increased IC₅₀ concentration. To be a significant change in affinity it is often stated that the difference in IC₅₀ should be at least 10-fold.

| **Table 1** | | |
|---|---|---|
| **Sample** | **IC₅₀ (nM)** | **IC₅₀ (relative rituximab)** |
| Rituximab | 26 | 1.0 |
| 1.6 1033-rituximab⁽¹⁾ | 106 | 4.1 |
| 2.4 1033-rituximab | 100 | 3.8 |
| 3.4 1033-rituximab | 350 | 13.5 |
| 4.6 1033-rituximab | 440 | 16.9 |
| Human IgG | No inhibition | -- |

| | | |
|---|---|---|
| (1) 1.6 1033-rituximab denotes 1033-rituximab conjugated with 1.6 MitraTag/rituximab | | |

A slight decrease in affinity was seen for the 1.6-and 2.4 1033-rituximab conjugates, whereas the decrease for the 3.4- and 4.6- 1033-rituximab conjugates was slightly above ten-fold relative to the IC50 concentration of Rituximab. The affinity for the 3.4- and 4.6-1033-rituximab conjugates is probably still high enough to obtain a proper tumour uptake in patients.

It has been shown in clinical studies that a tenfold difference in affinity does not result in any significant difference in tumour uptake (ref. 6). Therefore, it was concluded that conjugation of Rituximab with up to 3-4 conjugates per antibody would not diminish the binding properties of the antibody *in vivo.*

### Example 6 - Pharmacokinetics of MitraTag-1033 conjugates of rituximab.

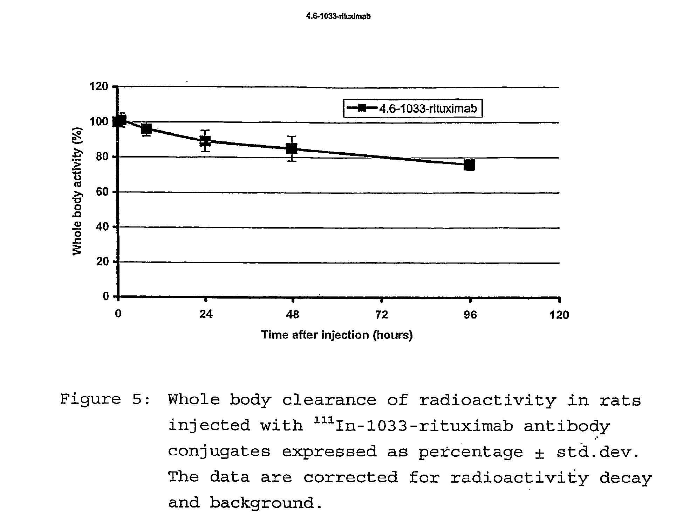

Rats of the Spraque Dawley strain were injected intravenously with approximately 50 µg of 1033-rituximab (4.6 1033 moieties per antibody) labelled with 3 - 4 MBq ¹¹¹Indium mixed with 1.2 mg/rat of a non-conjugated Rituximab. Whole body (WB) imaging was performed using a scintillation camera (General Electric 400T, GE, Milwaukee, WI, USA) equipped with a medium-energy collimator. Images were stored and analysed with Nuclear MAC 2.7 software. From images, the total number of counts in the entire body were obtained. After radioactivity decay correction and background subtraction, the counts were used for the calculation of activity retention (%) in the body. See Fig. 5.

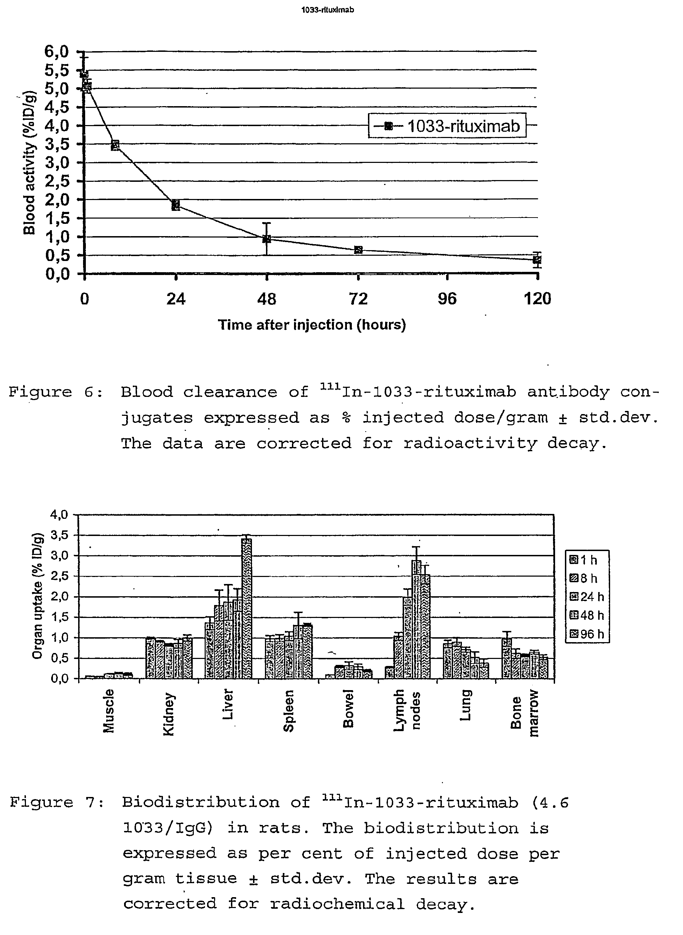

To define pharmacokinetics of ¹¹¹In-1033-rituximab and compare it with ¹¹¹In-DOTA-hMn14, about 0.2 ml blood was obtained from the periorbital venous plexa on the following occasions: 10 min, 1, 8, 24, 48 and 96 hours post injection. The radioactivity was measured in an automatic NaI(Tl) scintillation well counter and expressed in percent of injected activity per gram blood (%/g) corrected for ¹¹¹In decay (Fig. 6).

### Example 7 - Biodistribution of conjugates to organs and tissues.

At dissections, performed after 1, 8, 24, 48, and 96 hours post injection, organs and tissues of interest were removed, weighed and measured for radioactivity content. The radioactivity was measured in an automatic NaI (Tl) scintillation well counter, and the counts were corrected for decay. The distribution of the injected activity is shown in Fig. 7, and Table 2.

| **Table 2** | **Uptake of ¹¹¹In-1033-rituximab (% injected dose/g)** | | | | |
|---|---|---|---|---|---|
| **Tissue** | **1 h** | **8 h** | **24 h** | **48 h** | **96 h** |
| **Muscle** | 0.06 | 0.06 | 0.12 | 0.13 | 0.11 |
| **Kidney** | 0.98 | 0.91 | 0.83 | 0.86 | 1.00 |
| **Liver** | 1.22 | 1.79 | 1.86 | 1.92 | 3.42 |
| **Spleen** | 1.02 | 0.99 | 1.04 | 1.30 | 1.31 |
| **Bowel** | 0.10 | 0.29 | 0.32 | 0.29 | 0.20 |
| **Lymph nodes** | 0.26 | 1.03 | 1.99 | 2.88 | 2.54 |
| **Lung** | 0.74 | 0.89 | 0.71 | 0.52 | 0.38 |
| **Bone marrow** | 0.79 | 0.62 | 0.57 | 0.65 | 0.52 |

### Example 8 - In vivo stability of the radiolabelled MitraTag-1033 antibody conjugates.

The stability of the MitraTag^{™}-1033 moiety *in vivo* was determined by analysing the percentage of radioactivity in blood binding to Avidin-microcolumns.

About 0.1 ml blood was obtained from the periorbital venous plexa on following occasions: 1, 8, 24, 48, and 96 hours post injection. 50 µl blood was applied to a micro-column with Avidin-agarose (0.3 ml adsorbent). After incubation for 10 minutes, the unbound radioactivity was washed off the column. The radioactivity in the column and the collected washing fluid was measured in an automatic NaI(Tl) scintillation well counter and the bound fraction was expressed in percent of the total radioactivity applied to the column.

| **Time post injection (hours)** | **% Avidin-binding** | **Range (%)** | **Animals analysed** |
|---|---|---|---|
| **0** | 99.2 | 99.1 99.3 | - |
| **1** | 99.4 | 99.4 - 99.5 | 3 |
| **8** | 99.4 | 99.4 - 99.4 | 3 |
| **24** | 99.3 | 99.2 - 99.4 | 2 |
| **48** | 99.1 | 98.9 - 99.3 | 3 |
| **96** | 98.5 | 97.7 - 99.1 | 3 |

Sample 0 is on the conjugate to be injected.

During the period studied, no reduction of the binding to avidin of the radioactivity present in blood could be detected.

Therefore, it was concluded that the linkage between biotin and the DOTA chelate is stable in blood circulation up to 96 hours post injection.

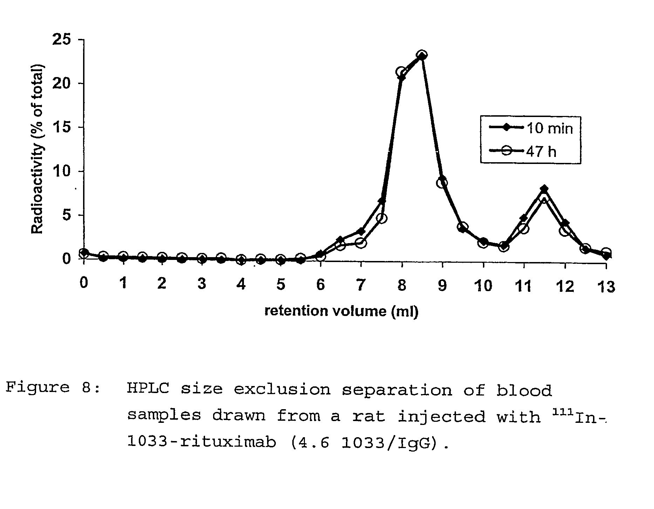

When plasma was separated on a HPLC size exclusion column, no significant change in size distribution could be seen when a 10 min sample was compared with a 47 hour sample (Fig. 8).

### Example 9 - Treatment regime in B-cell lymphoma according to the most preferred embodiment of the invention.

The treatment regime can be separated in the following events:
- All patients will receive a dose of 250 mg/m² rituximab one week prior to therapy (day -7) in order to eliminate the circulating B-cells, immediately followed by a diagnostic dose of 50-150 MBq (1.5-4 mCi) ¹¹¹In-1033-rituximab.
- On day 0 all patients will receive 250 mg/m2 rituximab immediately followed by a therapeutic dose of ⁹⁰Y-1033-rituximab (>10MBq/kg bodyweight). Patients may, optionally, be administered a dose of 150-250 MBq (4-7 mCi) ¹¹¹In-1033-rituximab, which will be used for imaging for dosimetry.
- On day 1 or 2, patients are treated with Mitradep^{®}, allowing 3 blood volumes to pass the Mitradep^{®} device.

## Claims

1. A medical agent comprising on average 1.5 to 3.5 reagents conjugated to an anti-CD20 antibody, wherein each reagent is 3-(13'-thioureabenzyl-(DOTA) trioxadi-amine-1-(13"-biotin-Asp-OH) trioxamine-5-isothio-cyanato-aminoisophtalate, and wherein the anti-CD20 antibody is rituximab.

2. The medical agent according to any one of the preceding claims, wherein it is provided with Y-90 or In-III.

3. A kit for extracorporeal elimination or at least reduction of the concentration of a non-tissue bound therapeutic or diagnostic medical agent as defined in any one of claims 1 or 2 in the plasma or whole blood of a mammalian host, said kit comprising
a) the medical agent, and
b) an extracorporeal device comprising an immobilised receptor to which the biotin molecule in the medical agent adheres.

## Patentansprüche

1. Medizinisches Mittel, welches im Mittel 1,5 bis 3,5 Reagenzien enthält, die an einen Anti-CD20-Antikörper konjugiert sind, wobei es sich bei dem Reagens jeweils um 3-(13'-Thioharnstoffbenzyl-(DOTA)trioxadiamin-1-(13"-biotin-Asp-OH)trioxamin-5-isothiocyanatoaminoisophthalat handelt, und wobei es sich bei dem Anti-CD20-Antikörper um Rituximab handelt.

2. Medizinisches Mittel nach Anspruch 1, welches mit Y-90 oder In-III bereitgestellt wird.

3. Kit zur extrakorporalen Eliminierung oder zumindest zur Verringerung der Konzentration eines nicht gewebegebundenen therapeutischen oder diagnostischen medizinischen Mittels nach Anspruch 1 oder 2, im Plasma oder Vollblut eines menschlichen oder Säugetier-Wirtes, wobei der Kit das Folgende umfasst:
a) das medizinische Mittel und
b) eine extrakorporale Vorrichtung, welche einen immobilisierten Rezeptor umfasst, an welchem das Biotinmolekül in dem medizinischen Mittel haftet.

## Revendications

1. Agent médical comprenant en moyenne 1,5 à 3,5 réactifs conjugués à un anticorps anti-CD20, dans lequel chaque réactif est 3-(13'-thiouréebenzyl-(DOTA)trioxadiamine-1-(13"-biotin-Asp-OH)trioxamine-5-isothio-cyanato-aminoisophtalate, et dans lequel l'anticorps anti-CD20 est le rituximab.

2. Agent médical selon l'une quelconque des revendications précédentes, dans lequel il est fourni avec Y-90 ou In-III.

3. Kit d'élimination extracorporelle ou au moins de réduction de la concentration d'un agent médical thérapeutique ou de diagnostic non lié aux tissus tel que défini dans l'une quelconque des revendications 1 ou 2 dans le plasma ou le sang total d'un hôte mammifère, ledit kit comprenant
a) l'agent médical, et
b) un dispositif extracorporel comprenant un récepteur immobilisé auquel adhère la molécule de biotine dans l'agent médical.
